(19) Europäisches Patentamt
European Patent Office
Office européen des brevets

(11) Publication number: **0 402 717 A1**

(12) **EUROPEAN PATENT APPLICATION**

(21) Application number: 90110452.1

(22) Date of filing: 01.06.90

(51) Int. Cl.5: C07D 401/06, C07D 401/04, C07D 403/04, C07D 403/06, C07D 239/06, A01N 43/54

(30) Priority: 14.06.89 JP 149696/89

(43) Date of publication of application: 19.12.90 Bulletin 90/51

(84) Designated Contracting States: BE CH DE DK ES FR GB IT LI NL

(71) Applicant: NIHON TOKUSHU NOYAKU SEIZO K.K.
7-1, Nihonbashi Honcho 2-Chome Chuo-ku
Tokyo(JP)

(72) Inventor: Shiokawa, Kozo
2-23-30 Shukugawara, Tama-ku
Kawasaki-shi, Kanagawa-ken(JP)
Inventor: Tsuboi, Shinichi
3-26-1, Hirayama
Hino-shi, Tokyo(JP)
Inventor: Moriya, Koichi
3-19-5 Ekiminami-machi
Oyama-shi, Tochigi(JP)
Inventor: Hattori, Yumi
598 Kobiki-machi
Hachioji-shi, Tokyo(JP)
Inventor: Honda, Ikuro
3-17-7, Minami-machi
Tanashi-shi, Tokyo(JP)
Inventor: Shibuya, Katsuhiko
39-15, Namiki-cho
Hachioju-shi, Tokyo(JP)

(74) Representative: Ernst, Hilmar, Dr. et al
Bayer AG Konzernverwaltung RP
Patentabteilung
D-5090 Leverkusen 1, Bayerwerk(DE)

(54) **5-nitro-tetrahydropyrimidines and insecticides containing them for agricultural uses.**

(57) The present invention relates to novel 5-nitro-tetrahydropyrimidines, processes for the preparation thereof and their use as insecticides.

The novel compounds can be characterized by the general formula (I)

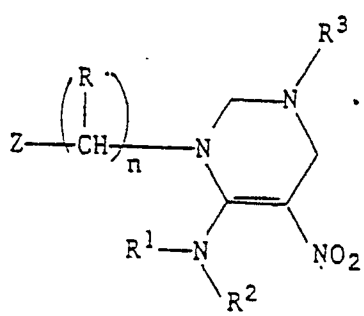

(I)

wherein

Z represents a substituted phenyl group or an optionally substituted five-membered or six-membered heterocyclic group comprising at least one hetero atom selected from the group consisting of nitrogen atom, oxygen atom and sulfur atom,

EP 0 402 717 A1

R represents hydrogen atom or a lower alkyl group,
$R^1$ and $R^2$ each represent hydrogen atom, an optionally substituted lower alkyl group, a lower alkenyl group, a lower alkynyl group, an optionally substituted phenyl group, an optionally substituted benzyl group,
$R^3$ represents a hydrogen atom, an optionally substituted alkyl group, a lower alkenyl group, a lower alkynyl group, a cycloalkyl group optionally substituted by a lower alkyl group, an optionally substituted phenyl group, an optionally substituted benzyl group, an optionally substituted heterocyclic group, and n represents 0 or 1.

## 5-NITRO-TETRAHYDROPYRIMIDINES AND INSECTICIDES CONTAINING THEM FOR AGRICULTURAL USES

The present invention relates to novel 5-nitro-tetrahydropyrimidines, processes for the preparation thereof, and their use in insecticidal compositions.

In a publication entitled Chem. Ber. vol. 119, pages 2208 to 2219, 1968 that was published before the filing date of the present application is disclosed a compound respresented by the general formula:

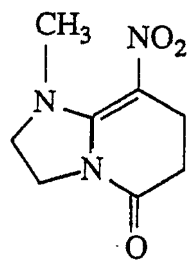

In a publication entitled "J. Chem. Soc. Perkin Trans. I", 1979, pages 2361 to 2363 is disclosed a compound represented by the formula

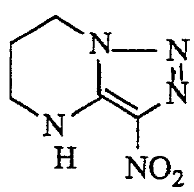

Further, a publication entitled "J. Heterocyclic Chem." vol. 17, page 1413, 1980 discloses the following compounds:

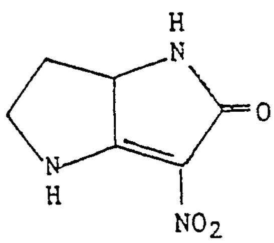

,

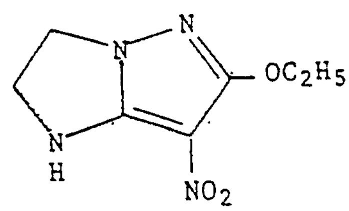

On the other hand, a publication entitled "Heterocycles", vol. 15, page 437, 1980 discloses a compound represented by the general formula:

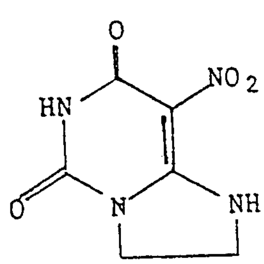

Similarly, "Yakugaku Zasshi" vol. 97, No. 3, pages 262 - 267, 1977 and vol. 97, No. 9, pages 1039 to 1045, 1977 each disclosed nitrogen-containing six-membered heterocyclic rings.

Japanese Patent Application Disclosure No. 3184-1989 filed by the same applicant as that of the

present application discloses a certain kind of nitro-substituted heterocyclic compounds that is stated to be useful as insecticide.

It has now be found that the novel 5-nitro-tetrahydropyrimidines having the following general formula (I):

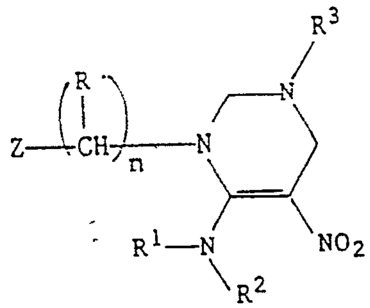

(I)

wherein

Z represents a substituted phenyl group or an optionally substituted five-membered or six-membered heterocyclic group comprising at least one hetero atom selected from the group consisting of nitrogen atom, oxygen atom and sulfur atom,

R represents hydrogen atom or a lower alkyl group,

$R^1$ and $R^2$ each represent hydrogen atom, an optionally substituted lower alkyl group, a lower alkenyl group, a lower alkynyl group, an optionally substituted phenyl group or an optionally substituted benzyl group,

$R^3$ represents a hydrogen atom, an optionally substituted alkyl group, a lower alkenyl group, a lower alkynyl group, a cycloalkyl group optionally substituted by a lower alkyl group, an optionally substituted phenyl group, an optionally substituted benzyl group, an optionally substituted heterocyclic group, and n represents 0 or 1.

The compounds represented by the general formula (I) according to the present invention can be prepared, for example, by the following processes:

Process a):

A process for the preparation of 5-nitro-tetrahydropyrimidines represented by the above-mentioned general formula (I) according to the present invention, which comprises reacting a compound represented by the general formula:

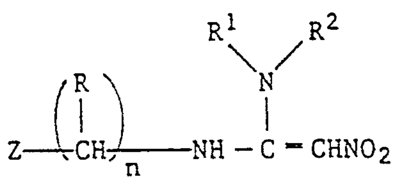

(II)

wherein Z, R, $R^1$ and $R^2$ have the same meanings as mentioned before, with formaldehyde and a compound represented by the general formula:

$NH_2$-$R^3$ (III)

wherein $R^3$ has the same meaning as mentioned before.

Process b):

A process for the preparation of 5-nitro-tetrahydropyrimidines represented by the general formula (I) according to the present invention, which comprises reacting a compound represented by the general formula:

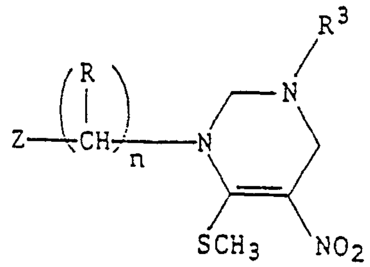

(IV)

wherein Z, R, $R^3$ and n have the same meanings as mentioned before, with a compound represented by the general formula:

$NHR^1R^2$ (V)

wherein $R^1$ and $R^2$ have the same meanings as mentioned above.

The 5-nitro-tetrahydropyrimidines represented by the general formula (I) according to the present invention exhibit a strong insecticidal activity.

Surprisingly, the compounds represented by the general formula (I) according to the present invention exhibit a substantially better insecticidal activity than that exhibited by the compounds which were disclosed in the above-mentioned articles "Chem. Ber.", vol. 119, 2208 to 2219, 1968 and "Heterocycles", vol. 15, page 437, 1980.

Referring to the general formula (I) according to the present invention, the respective symbols preferably have the following meanings:

Z represents a phenyl group, substituted by halogen, a six-membered heterocyclic group comprising one to two nitrogen atoms and which is optionally substituted by a halogen atom or a lower alkyl group, or a five-membered heterocyclic group comprising one or two nitrogen atoms and either of oxygen atom or sulfur atom and which is optionally substituted by a halogen atom or a lower alkyl group,

R represents hydrogen atom or methyl group,

$R^1$ and $R^2$ each represent hydrogen atom, or a lower alkyl group, optionally substituted by halogen, cyano, trifluoromethyl or by an optionally substituted heterocyclic group, allyl group, propargyl group, or phenyl group, optionally substituted by halogen or cyano, a benzyl group, optionally substituted by halogen, 3-pyridylmethyl group, or 2-chloro-5-pyridylmethyl group,

$R^3$ represents a $C_1$-$C_{14}$ alkyl group, allyl group, propargyl group, a $C_5$-$C_6$ cycloalkyl group, optionally substituted by methyl, a phenyl group, optionally substituted by halogen or lower alkyl, a benzyl group, optionally substituted by halogen or lower alkyl or 2 chloro-5-pyridylmethyl group, and

n represents 0 or 1.

In the above definitions lower alkyl group stands for alkyl having 1 to 6 carbon atoms and preferably 1 to 4 carbon atoms, lower alkenyl group stands for alkenyl having 2 to 6 and preferably 3 to 5 carbon atoms and lower alkynyl group stands for alkynyl having 2 to 6 and preferably 3 to 5 carbon atoms.

Further, referring to the general formula (I), the respective symbols most preferably have the following meanings:

Z represents the 3-pyridyl group or the 2-chloro-5-pyridyl group,

R represents a hydrogen atom,

$R^1$ and $R^2$ each represents hydrogen atom or methyl group,

$R^3$ represents a $C_1$-$C_{12}$ alkyl group, allyl group, cyclohexyl group, a phenyl group, optionally substituted by lower alkyl, a benzyl group, optionally substituted by fluorine or chlorine, or 2-chloro-5-pyridylmethyl group, and

n represents 1.

As individual examples of the compounds represented by the general formula (1) according to the present invention the following ones may be mentioned:

3-(2-chloro-5-pyridylmethyl)-1-methyl-4-methylamino-5-nitro-1,2,3,6-tetrahydropyrimidine,

3-(2-chloro-5-pyridylmethyl)-1-isopropyl-4-methylamino-5-nitro-1,2,3,6-tetrahydropyrimidine, and

3-(2-chloro-5-pyridylmethyl)-1-dodecyl-4-methylamino-5-nitro-1,2,3,6-tetrahydropyrimidine.

In the process a), if use is made, as starting material, of 1-(2-chloro-5-pyridylmethylamino)-1-methylamino-2-nitroethylene, methylamine and formaldehyde, for example, the reaction can be expressed as follows:

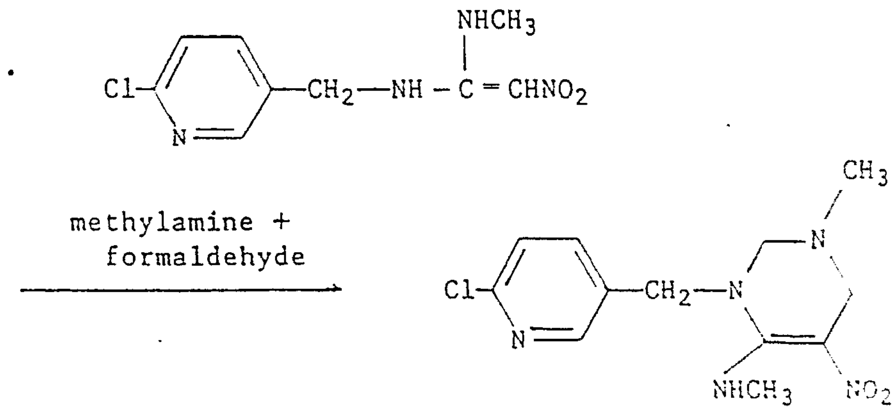


In the process b), if use is made, as starting material, of 3-(2-chloro-5-pyridylmethyl)-1-methyl-4-methylthio-5-nitro-1,2,3,6-tetrahydropyrimidine and methylamine, the reaction can be expressed as follows:

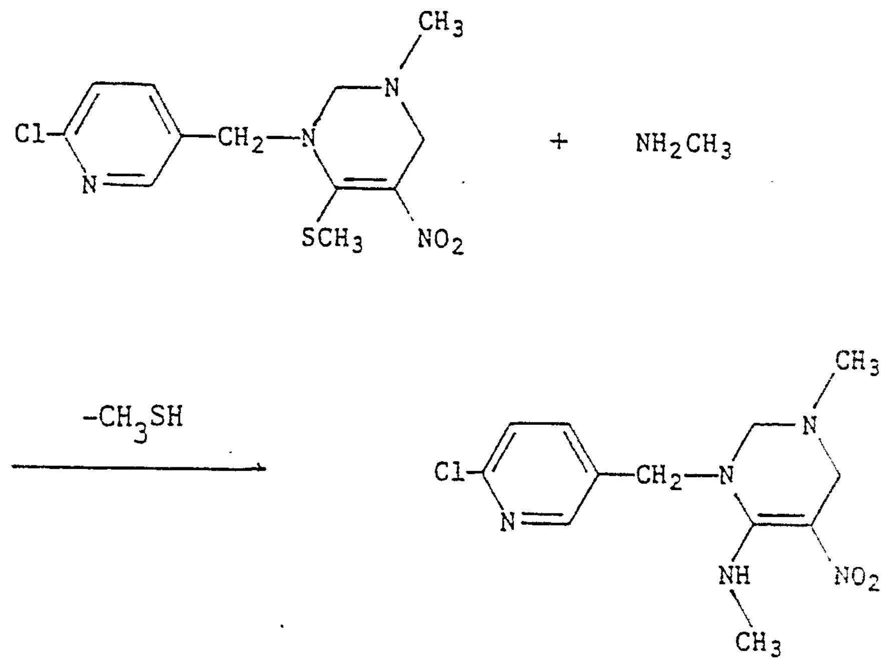


The material compounds represented by the general formula (II) and employed in the above-mentioned process a) are what were defined before to have the substituents defined under symbols Z, R, $R^1$, and $R^2$.

Further, the symbols Z, R, $R^1$ and $R^2$ in the general formula (II) are what were defined before under the stated preferable substituents.

The compounds represented by the general formula (II) include those described in and known from Japanese Patent Application No. 299419-1988 and others and may be individually exemplified by 1-(2-chloro-5-pyridylmethylamino)-1-methylamino-2-nitroethylene, for example.

The material compounds represented by the general formula (III) and employed in the process a) are what were defined before to have a substituent defined under symbol $R^3$ and especially the preferable ones.

The compounds represented by the general formula (III) are well known in the field of organic chemistry and, as individual examples the following compounds may be exemplified: methylamine, isopropylamine and dodecylamine, for example.

Referring to the material compounds represented by the general formula (IV) and employed in the process b), the symbols Z, R, $R^3$ and n are what were defined before and especially under the preferable ones.

The compounds represented by the general formula (IV) can be obtained, for example, by the following

process:

A process for the preparation of a compound represented by the above-mentioned general formula (IV), which comprises reacting a compound represented by the general formula

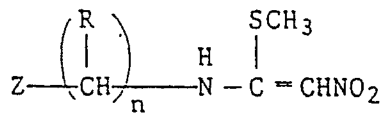

(VI)

wherein Z, R and n have the same meanings as mentioned before, with formaldehyde and a compound represented by the afore-mentioned general formula (III).

The above-mentioned compounds represented by the general formula (VI) are disclosed in Japanese Patent Application No. 299419-1988 and may be concretely exemplified by 1-(2-chloro-5-pyridylmethylamino)-1-methylthio-2-nitroethylene.

In carrying out the process a) mentioned above, any inert solvent may be used as diluent.

Examples of such diluents are water; aliphatic, cycloaliphatic and aromatic, optionally chlorinated, hydrocarbons, such as hexane, cyclohexane, petroleum ether, ligroin, benzene, toluene, xylene, methylene chloride, chloroform, carbon tetrachloride, ethylene chloride, trichloroethylene, chlorobenzene, and the like; ethers such as diethyl ether, methyl ethyl ether, di-isopropyl ether, dibutyl ether, propylene oxide, dioxane, tetrahydrofuran and the like; nitriles such as acetonitrile, propionitrile, acrylonitrile and the like; alcohols such as methanol, ethanol, iso-propanol, butanol, ethylene glycol and the like; esters such as ethyl acetate, amyl acetate and the like; acid amides such as dimethyl formamide, dimethyl acetamide and the like; and sulfones and sulfoxides such as dimethyl sulfoxide, sulfolane and the like; and bases, for example, pyridine, etc.

In the process a), the reaction temperature can be varied within a wide range. In general, the reaction is carried out at a temperature from about 0 to about 150 °C, preferably from about 50 to about 90 °C. In general, the reaction is allowed to proceed under normal pressure, although it is also possible to employ a higher or lower pressure.

When the process a) according to the present invention is carried out, use is made, for instance, of 1 to 5 moles of the compound of the general formula (III) and 2 to 10 moles of formaldehyde, per 1 mole of the compound of the general formula (II), for example, in the presence of ethanol to obtain the aimed compound.

In carrying out the process b), use is made, as suitable diluent, of any inert solvents.

Examples of such diluents are water; aliphatic, cycloaliphatic and aromatic, optionally chlorinated, hydrocarbons, such as hexane, cyclohexane, petroleum ether, ligroin, benzene, toluene, xylene, methylene chloride, chloroform, carbon tetrachloride, ethylene chloride, trichloroethylene, chlorobenzene, and the like; ethers such as diethyl ether, methyl ethyl ether, di-isopropyl ether, dibutyl ether, propylene oxide, dioxane, tetrahydrofuran and the like; nitriles such as acetonitrile, propionitrile, acrylonitrile and the like; alcohols such as methanol, ethanol, iso-propanol, butanol, ethylene glycol and the like; esters such as ethyl acetate, amyl acetate and the like; acid amides such as dimethyl formamide, dimethyl acetamide and the like; and sulfones and sulfoxides such as dimethyl sulfoxide, sulfolane and the like; and bases, for example, pyridine, etc.

In the process b), the reaction temperature can be varied within a wide range. In general, the reaction is carried out at a temperature from about 0 to about 150 °C, preferably about 20 to about 80 °C. In general, the reaction is allowed to proceed under normal pressure, although it is also possible to employ a higher or lower pressure.

When the process b) according to the present invention is carried out, use is made, for instance, of 1 to 5 moles of the compound represented by the general formula (V) per 1 mole of the compound represented by the general formula (IV), for example, in the presence of ethanol as solvent to obtain the aimed compound.

The compounds represented by the general formula (I) according to the present invention exhibit a strong insecticidal activity, therefore they can be used as insecticides.

The active compounds according to the present invention exhibit no phytotoxity to any useful plants under cultivation but they demonstrate a stable pest-control effect, and can be used for combating various noxious pests, especially insects, including imbibing insects, biting insects, and other plant-parasitic pests, and other noxious pests in habiting in stored products and materials and harmful in the hygiene field.

Such pests and insects as mentioned above may be exemplified as follows:

From the order of Coleoptera, e.g. Callosobruchus chinensis, Sitophilus zeamais, Tribolium castaneum, Epilachna vigintioctomaculata, Agriotes fuscicollis, Anomala rufocuprea, Leptinotarsa decemlineata, Diabrotica spp., Monochamus alternatus, Lissorhoptrus oryzophilus, Lyctus brunneus; from the order of Lepidoptera, e.g. Lymantria dispar, Malacosoma neustria, Pieris rapae, Spodoptera litura, Mamestra brassicae, Chilo suppressalis, Pyrausta nubilalis, Ephestia cautella, Adoxophyes orana, Carpocapsa pomonella, Agrotis fucosa, Galleria mellonella, Plutella maculipennis, Phyllocnistis citrella; from the order of Hemiptera, e.g. Nephotettix cincticeps, Nilaparvata lugens, Pseudococcus comstocki, Unaspis yanonensis, Myzus persicae, Aphis pomi, Aphis gossypii, Rhopalosiphum pseudobrassicae, Stephanitis nashi, Nezara spp., Cimex lectularius Trialeurodes vaporariorum, Psylla spp.; from the order of Orthoptera, e.g. Blatella germanica, Periplaneta americana, Gryllotalpa africana, Locusta migratoria migratoriodes; from the order of Isoptera, e.g. Deucotermes speratus, Coptotermes formosanus; from the order of Diptera, e.g. Musca domestica, Aedes aegypti, Hylemya platura; Culex pipiens, Anopheles slnensis, Culex tritaeniorhynchus.

As mites may be mentioned, for example, Tetranychus telarius, Tetranychus urticae, Panonychus citri, Aculops pelekassi, Tarsonemus spp., and like.

As nematodes may be mentioned Meloidogyne incognita, Bursaphelenchus lignicolus Mamiya et Kiyohara, Aphelenchoides besseyi, Heterodera glycines, Pratylenchusspp., and like.

Furthermore, in the field of veterinary medicine, the novel compounds of the present invention can effectively be employed for combating a variety of noxious animal-parasitic pests (internal- and external-parasitic pests), e.g. mites, insects and nematodes. Such animal-parasitic pests may be exemplified as follows:

From the class of Insecta, e.g. Gastrophilus spp., Stomoxys spp., Tricodectes spp., Rhodnius spp., Ctenocephalides canis and the like.

As mites may be mentioned Ornithodoros spp., Ixodes spp., Boophilus spp., and like.

According to the present invention, the term "insecticides" may mean whatever substance which exhibits pesticidal action against all the insects and pests mentioned above.

The active compounds of the present invention can be prepared into the customary formulations, such as solutions, emulsions, suspensions, powders, foams, pastes, granules, aerosols, natural and synthetic materials impregnated with active compounds, micro-capsules, coating compositions for use on seed, and formulations used with burning equipment, such as fumigating cartridges, fumigating cans and fumigating coils, as well as ULV cold mist and warm mist formulations.

These formulations may be produced in a known manner, for example, by mixing the active compounds with extenders, that is to say liquid or liquefied gaseous or solid diluents or carriers, optionally with the use of surface-active agents, that is to say emulsifying agents, dispersing agents, and/or foam-forming agents. In the case of using water as an extender, organic solvents can, for example, also be used as auxiliary solvents.

As liquid diluents or carriers can be mentioned, for example, aromatic hydrocarbons, such as xylene, toluene and alkyl naphthalenes, chlorinated aromatic or chlorinated aliphatic hydrocarbons, such as chlorobenzenes, chloroethylenes and methylene chloride, aliphatic or alicyclic hydrocarbons, such as cyclohexane or paraffins, for example, mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethylsulfoxide, as well as water.

By liquefied gaseous diluents or carriers are meant liquids which are gaseous at normal temperature and under normal pressure, for example, aerosol propellants, such as halogenated hydrocarbons as well as butane, propane, nitrogen and carbon dioxide.

As solid diluents there may be used ground natural minerals, such as kaolins, clays, talc, chalk, quartz, attapulgite, montmorillonite or diatomaceus earth, and ground synthetic minerals, such as highly-dispersed silicic acid, alumina and silicates.

As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used nonionic and anionic emulsifiers, such as polyoxyethylene-fatty acid esters, polyoxyethylene-fatty alcohol ethers, for example, alkylaryl polyglycol ethers, alkylsulfonates, alkylsulfates, arylsulfonates as well as albumin hydrolysis products. Dispersing agents include, for example, lignin sulfite waste liquors and methyl cellulose.

Adhesives such as carboxymethyl cellulose and natural and synthetic polymers, (such as gum arabic, polyvinyl alcohol and polyvinyl acetate) can be used in the formulations in the form of powders, granules or emulsifiable concentration.

It is possible to use colorants such as inorganic pigments, for example: iron oxide, titanium oxide and Prussian Blue, and organic dyestuffs, such as alizarin dyestuffs, azo dyestuffs or metal phthalocyanine dyestuffs, and trace elements, such as salts of iron, manganese, boron, copper, cobalt, molybdenum and zinc.

The formulations, in general, contain from 0.1 to 95 percent by weight of active compound, preferably from 0.5 to 90 percent by weight.

The active compounds of the present invention having the formula (I) can be used as such, as their commercially useful formulations or as the use forms prepared therefrom, optionally together with other active agents such as, for example, insecticides, poisonous baits, bactericides, acaricides, nematicides, fungicides, growth regulators or herbicides. Examples of the pesticides include, e.g. those derived from organic phosphosphates, carbamates, carboxylates, chlorohydrocarbons, and antibiotic products.

Furthermore, the active compound of the present invention having the formula (I) can be present as a mixture with a synergist in a formulation or a use form, of the type that is commercially useful. The term "synergist" denotes a compound which is not active in itself, but promotes the action of an active compound. The content of the active compounds having the general formula (I) of the present invention in the formulations of the types that are commercially available can vary within wide ranges. The active compound concentration of the formulation for use is, for example, from 0.0000001 to 100 percent by weight, preferably from 0.00001 to 1 percent by weight.

The compounds of the formula (I) may be employed in a customary manner appropriate for a particular use form.

When used against pests harmful to health and pests to stored products, the active compounds are distinguished by an excellent residual activity on wood and soil as well as a good stability to alkali on limed substrates.

The invention will be further illustrated by way of examples. However, it should be noted that the scope of the invention is not limited only to that of examples.

Examples of Preparation:

Example 1

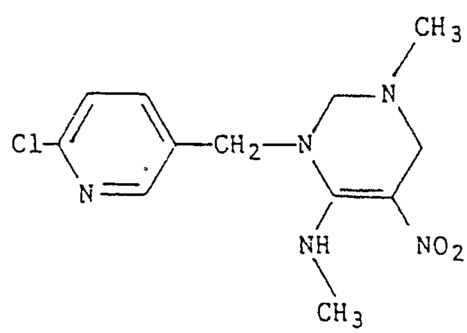

A liquid mixture consisting of 1-(2-chloro-5-pyridylmethylamino)-1-methylamino-2-nitroethylene (1.0 g), a methanol solution of methylamine (40%, 0.35 g), an aqueous solution of formaldehyde (37%, 0.7 g) and ethanol (40 ml) was refluxed under heating for two hours. The solvent was distilled off from the liquid mixture under reduced pressure followed by refining of the resulting residue with the use of a column chromatography (elute: ethanol : chloroform = 1 : 10) to obtain the aimed 3-(2-chloro-5-pyridylmethyl)-1-methyl-4-methylamino-5-nitro-1,2,3,6-tetrahydropyrimidine (0.5 g) having a melting point in the range of from 123 to 124° C.

Example 2

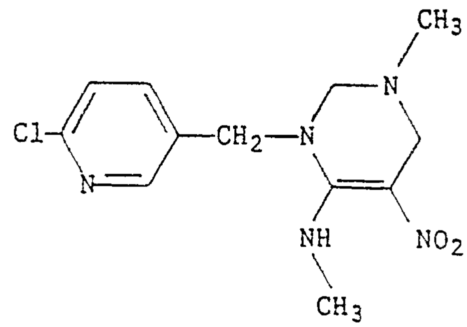

A mixture consisting of 3-(2-chloro-5-pyridylmethyl-1-methyl-4-methylthio-5-nitro-1,2,3,6-tetrahydropyrimidine (1.0 g), a methanol solution of methylamine (40%, 1.2 g), and ethanol (40 ml) was heated at 50° C for 24 hours. Under reduced pressure, the solvent was distilled off from the mixture, followed by refining of the resulting residue with the aid of column chromatography (elute: ethanol : chloroform = 1 : 10) to obtain the aimed 3-(2-chloro-5-pyridylmethyl)-1-methyl-4-methylamino-5-nitro-1,2,3,6-tetrahydropyrimidine (0.17 g) having a melting point in the range of from 123 to 124° C.

The compounds according to the present invention which can be prepared according to processes similar to those employed in the above-mentioned Examples 1 and 2 are shown in the following Table 1, including those which were prepared in the foregoing Examples 1 and 2.

Table 1

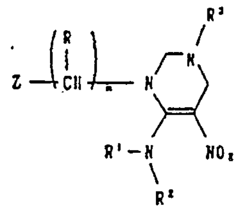

| Comp. No. | Z | R | $R^1$ | $R^2$ | n | $R^3$ | Melting point or refractive index |
|---|---|---|---|---|---|---|---|
| 1 | 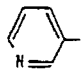 | — | $CH_3$ | H | 0 | $CH_3$ | |
| 2 | 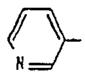 | H | $CH_3$ | H | 1 | $CH_3$ | mp. 127~132℃ |
| 3 | 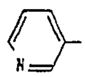 | H | $CH_3$ | $CH_3$ | 1 | $CH_3$ | |
| 4 | 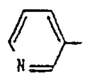 | H | $CH_3$ | H | 1 | $CH_2CH_3$ | |
| 5 | 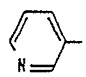 | H | $CH_3$ | H | 1 | $CH(CH_3)_2$ | |
| 6 | 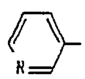 | $CH_3$ | $CH_3$ | H | 1 | $CH_3$ | |
| 7 | 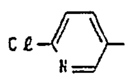 | — | $CH_3$ | H | 0 | $CH_3$ | |
| 8 | 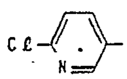 | H | $CH_3$ | H | 1 | $CH_3$ | mp. 123~124℃ |
| 9 | 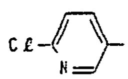 | H | $CH_3$ | $CH_3$ | 1 | $CH_3$ | |
| 10 | 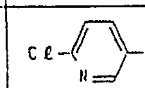 | $CH_3$ | $CH_3$ | H | 1 | $CH_3$ | |
| 11 | 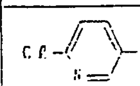 | H | $CH_3$ | H | 1 | $CH_2CH_3$ | |

Table 1 (continued)

| Comp No. | Z | R | $R^1$ | $R^2$ | n | $R^3$ | Melting point or refractive index |
|---|---|---|---|---|---|---|---|
| 12 | 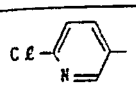 | — | $CH_3$ | H | 0 | $CH_2CH_3$ | |
| 13 | 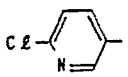 | H | $CH_3$ | $CH_3$ | 1 | $CH_2CH_3$ | |
| 14 | 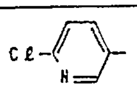 | H | $CH_3$ | H | 1 | $CH_2CH_2CH_3$ | mp. 154~158°C |
| 15 | 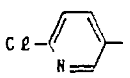 | H | $CH_3$ | H | 1 | $CH(CH_3)_2$ | mp. 132~136°C |
| 16 | 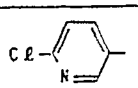 | H | $CH_3$ | $CH_3$ | 1 | $CH(CH_3)_2$ | |
| 17 | 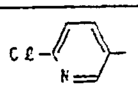 | H | $CH_3$ | H | 1 | $(CH_2)_3CH_3$ | |
| 18 | 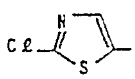 | H | $CH_3$ | H | 1 | $CH_3$ | |
| 19 | 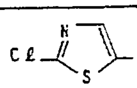 | H | $CH_3$ | $CH_3$ | 1 | $CH_3$ | |
| 20 | 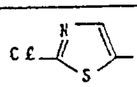 | H | $CH_3$ | H | 1 | $CH_2CH_3$ | |
| 21 | 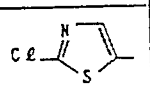 | H | $CH_3$ | H | 1 | $CH_2CH_2CH_3$ | |
| 22 | 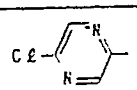 | H | $CH_3$ | H | 1 | $CH_3$ | |
| 23 | 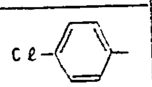 | H | $CH_3$ | H | 1 | $CH_3$ | |
| 24 | 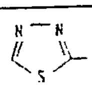 | H | $CH_3$ | H | 1 | $CH_3$ | |

Table 1 (continued)

| Comp. No. | Z | R | R¹ | R² | n | R³ | Melting point or refractive index |
|---|---|---|---|---|---|---|---|
| 25 | 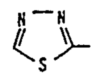 | H | $CH_3$ | $CH_3$ | 1 | $CH_3$ | |
| 26 | 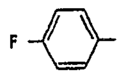 | H | $CH_3$ | $CH_3$ | 1 | $CH_2CH_3$ | |
| 27 | 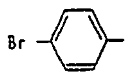 | — | $CH_3$ | H | 1 | $CH_3$ | |
| 28 | 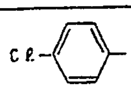 | — | $CH_3$ | H | 1 | $CH(CH_3)_2$ | |
| 29 | 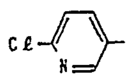 | H | $CH_3$ | H | 1 | $CH_2CH(CH_3)_2$ | |
| 30 | 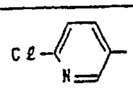 | H | $CH_3$ | H | 1 | $CH(CH_3)CH_2CH_3$ | |
| 31 | 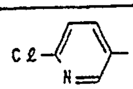 | H | $CH_3$ | H | 1 | $C(CH_3)_3$ | |
| 32 | 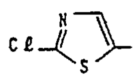 | H | $CH_3$ | $CH_3$ | 1 | $C(CH_3)_3$ | |
| 33 | 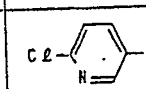 | H | 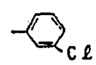 | H | 1 | $C(CH_3)_3$ | |
| 34 | 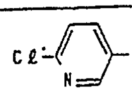 | H | $CH_2CN$ | H | 1 | $C(CH_3)_3$ | |
| 35 | 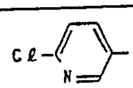 | H | $CH_3$ | H | 1 | $(CH_2)_4CH_3$ | |
| 36 | 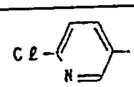 | H | $CH_3$ | H | 1 | $(CH_2)_5CH_3$ | |
| 37 | 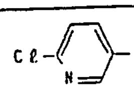 | H | $CH_3$ | H | 1 | $(CH_2)_6CH_3$ | |

Table 1 (continued)

| Comp. No. | Z | R | $R^1$ | $R^2$ | n | $R^3$ | Melting point or refractive index |
|---|---|---|---|---|---|---|---|
| 38 | 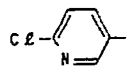 | H | $CH_3$ | H | 1 | $(CH_2)_7CH_3$ | |
| 39 | 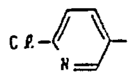 | H | $CH_3$ | H | 1 | $(CH_2)_8CH_3$ | |
| 40 | 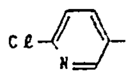 | H | $CH_3$ | H | 1 | $(CH_2)_9CH_3$ | |
| 41 | 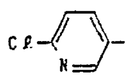 | H | $CH_3$ | H | 1 | $(CH_2)_{11}CH_3$ | mp. 111~113°C |
| 42 | 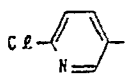 | H | $CH_3$ | $CH_3$ | 1 | $(CH_2)_{11}CH_3$ | |
| 43 | 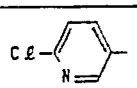 | H | $CH_2CH_3$ | H | 1 | $(CH_2)_{11}CH_3$ | |
| 44 | 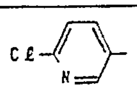 | H | $CH_2$ | H | 1 | $(CH_2)_{13}CH_3$ | |
| 45 | 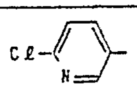 | H | $CH_2CN$ | H | 1 | $CH(CH_3)_2$ | |
| 46 | 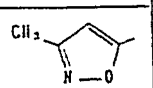 | H | $CH_2CF_3$ | H | 1 | $CH(CH_3)_2$ | |
| 47 | 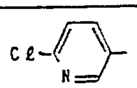 | H | $CH_2CH=CH_2$ | H | 1 | $(CH_2)_3CH_3$ | |
| 48 | 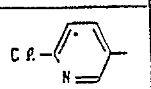 | H | $CH_2C≡CH$ | H | 1 | $(CH_2)_3CH_3$ | |
| 49 | 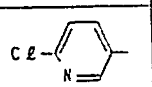 | H | 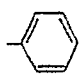 | H | 1 | $CH_3$ | |
| 50 | 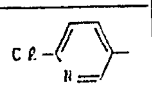 | H | 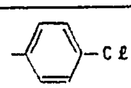 | H | 1 | $CH_3$ | |

Table 1 (continued)

| Comp. No. | Z | R | $R^1$ | $R^2$ | n | $R^3$ | Melting point or refractive index |
|---|---|---|---|---|---|---|---|
| 51 | 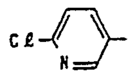 | H | 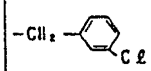 | H | 1 | $CH_3$ | |
| 52 | 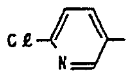 | H | 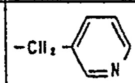 | H | 1 | $CH_3$ | |
| 53 | 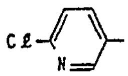 | H | 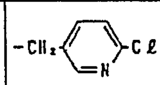 | H | 1 | $CH_3$ | |
| 54 | 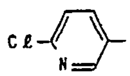 | H | $-CH_2CH_3$ | H | 1 | $CH_3$ | mp. 127~130℃ |
| 55 | 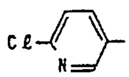 | H | $CH_3$ | H | 1 | $-CH_2CH=CH_2$ | mp. 145~150℃ |
| 56 | 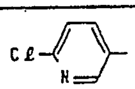 | H | $CH_3$ | H | 1 | $-CH_2C≡CH$ | |
| 57 | 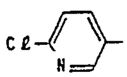 | H | $CH_3$ | H | 1 | 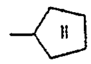 | |
| 58 | 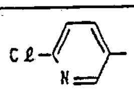 | H | $CH_3$ | H | 1 | 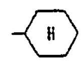 | $n_D^{20}$ 1.5940 |
| 59 | 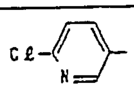 | H | $CH_3$ | H | 1 | 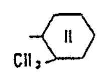 | |
| 60 | 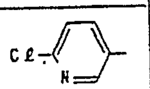 | H | $CH_3$ | H | 1 | 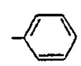 | |
| 61 | 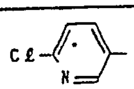 | H | $CH_3$ | H | 1 | 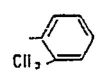 | |
| 62 | 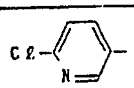 | H | $CH_3$ | H | 1 | 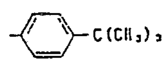 | mp. 155~160℃ |
| 63 | 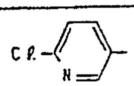 | H | $CH_3$ | H | 1 | 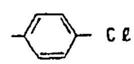 | |

Table 1 (continued)

| Comp. No. | Z | R | R¹ | R² | n | R³ | Melting point or refractive index |
|---|---|---|---|---|---|---|---|
| 64 | 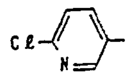 | H | $CH_3$ | H | 1 | 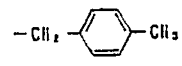 | |
| 65 | 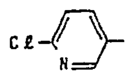 | H | $CH_3$ | H | 1 | 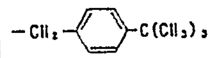 | |
| 66 | 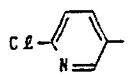 | H | $CH_3$ | H | 1 | 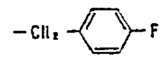 | mp. 167~169℃ |
| 67 | 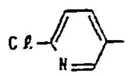 | H | $CH_3$ | H | 1 | 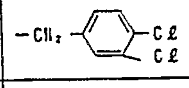 | $n_D^{20}$ 1.6215 |
| 68 | 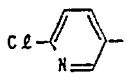 | H | $CH_3$ | H | 1 | 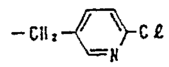 | mp. 170~172℃ |

Preparation of an immediate product:

Example 3

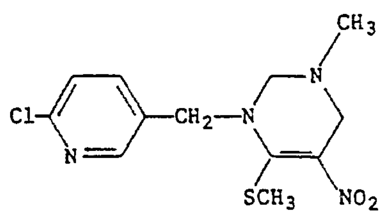

A mixture consisting of 1-(2-chloro-5-pyridylmethylamino)-1-methylthio-2-nitroethylene (2.0 g), a methanol solution of methylamine (40%, 0.7 g), an aqueous solution of formaldehyde (37%, 1.5 g) and ethanol (50 ml) was heated at 50 °C for two hours. The solvent was distilled off from the mixture under reduced pressure, followed by refining of the resulting residue with the use of a column chromatography (elute: ethanol : chloroform = 3 : 50) to obtain the aimed 3-(2-chloro-5-pyridylmethyl)-1-methyl-4-methylthio-5-nitro-1,2,3,6-tetrahydropyrimidine (1.0 g) having a refractive index of $n_D^{20}$ 1.5828.

Biotest Examples:

Comparative compounds

C-1:

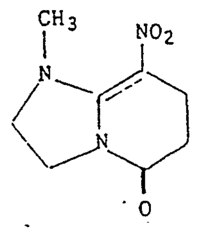

(disclosed by Chem. Ber., vol. 119, pp. 2208 - 2219, 1968)

C-2:

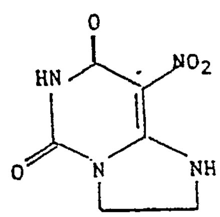

(disclosed in Heterocycles, vol. 15, p. 437, 1980)

Example 4

Test on Nephotettix cincticeps having resistance to organophosphorus agents:-

Preparation of a test chemical

Solvent: 3 parts by weight of xylene

Emulsifier: 1 part by weight of polyoxyethylene alkyl phenyl ether

To form a suitable preparation, 1 part by weight of the active compound was mixed with the aforesaid amount of the solvent containing the aforesaid amount of the emulsifier. The mixture was diluted with water to a predetermined concentration.

Testing method

Onto rice plants, about 10 cm tall, planted in pots each having a diameter of 12 cm was sprayed 10 ml per pot of the water-dilution of each active compound in a predetermined concentration prepared as above. The sprayed chemical as dried, and a wire net having a diameter of 7 cm and a height of 14 cm was put over each pot, and 30 female imagoes of Nephotettix cincticeps showing resistance to organophosphorus agents were released into the net. The pots were each placed in a constant temperature chamber and the number of dead insects was examined 2 days later, and the mortality was calculated.

The results are shown in Table 2.

Table 2

| Compound No. | Concentration of active compound (ppm) | Mortality (%) |
|---|---|---|
| 8 | 200 | 100 |
| 41 | 200 | 100 |
| 66 | 200 | 100 |
| Control | | |
| C-1 | 200 | 0 |
| C-2 | 200 | 0 |

Example 5

Test on plant hoppers

Test Method

A water dilution in a predetermined concentration of the active compound prepared as in Example 4 was sprayed onto rice plants, about 10 cm tall, grown in pots with a diameter of 12 cm in an amount of 10 ml per pot. The sprayed chemical was dried, and a wire net, 7 cm in diameter and 14 cm tall, was put over each of the pots. Thirty female imagoes of Nilaparvata lugens Stal of a strain which showed resistance to organophosphorus chemicals were released into the net. The pots were left to stand in a constant temperature chamber and the number of dead insects was examined two days later. The mortality was then calculated.

In the same way as above, the mortality was calculated on Sogatella furcifera Horvath and organophosphorus-resistant Laodelphax striatellus Fallen.

The results are shown in Table 3.

Table 3

| Compound No. | Concentration of actice compound (ppm) | Mortality (%) | | |
|---|---|---|---|---|
| | | Brown rice planthopper | Small brown planthopper | Whight bached rice planthopper |
| 8 | 500 | 100 | 100 | 100 |
| 41 | 500 | 100 | 100 | 100 |
| 86 | 500 | 100 | 100 | 100 |
| Control | | | | |
| C-1 | 500 | 0 | 0 | 0 |
| C-2 | 500 | 0 | 0 | 0 |

## Claims

1) 5-Nitro-tetrahydropyrimidines represented by the general formula

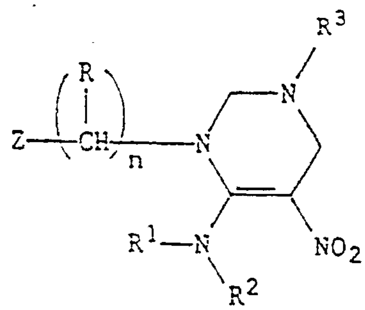

(I)

wherein
Z represents a substituted phenyl group or an optionally substituted five-membered or six-membered heterocyclic group comprising at least one hetero atom selected from the group consisting of nitrogen atom, oxygen atom and sulfur atom,
R represents hydrogen atom or a lower alkyl group,
$R^1$ and $R^2$ each represent hydrogen atom, an optionally substituted lower alkyl group, a lower alkenyl group, a lower alkynyl group, an optionally substituted phenyl group, an optionally substituted benzyl group,
$R^3$ represents a hydrogen atom, an optionally substituted alkyl group, a lower alkenyl group, a lower alkynyl group, a cycloalkyl group optionally substituted by a lower alkyl group, an optionally substituted phenyl group, an optionally substituted benzyl group, an optionally substituted heterocyclic group, and n represents 0 or 1.

2) 5-Nitro-tetrahydropyrimidines according to claim 1, wherein
Z represents a phenyl group substituted by halogen, a six-membered heterocyclic group comprising one to two nitrogen atoms which is optionally substituted by a halogen atom or a lower alkyl group, or a five-membered heterocyclic group comprising one or two nitrogen atoms and either of oxygen atom or sulfur atom and which is optionally substituted by a halogen atom or a lower alkyl group, R represents hydrogen atom or methyl group, $R^1$ and $R^2$ each represent hydrogen atom or a lower alkyl group, optionally substituted by halogen, cyano, trifluoromethyl or by an optionally substituted heterocyclic group, allyl group, propargyl group, a phenyl group, optionally substituted by halogen or cyano, a benzyl group, optionally substituted by halogen, 3-pyridylmethyl group, or 2-chloro-5-pyridylmethyl group, $R^3$ represents a $C_1$-$C_{14}$ alkyl group, allyl group, propargyl group, a $C_5$-$C_6$ cycloalkyl group, optionally substituted by methyl, a phenyl group, optionally substituted by halogen or lower alkyl, a benzyl group, optionally substituted by halogen or lower alkyl or 2-chloro-5-pyridylmethyl group, and
n represents 0 or 1.

3) 5-Nitro-tetrahydropyrimidines according to claim 1 or 2, wherein
Z represents the 3-pyridyl group or the 2-chloro-5-pyridyl group,
R represents a hydrogen atom,
$R^1$ and $R^2$ each represents a hydrogen atom or a methyl group,
$R^3$ represents a $C_1$-$C_{12}$ alkyl group, allyl group, cyclohexyl group, phenyl group, optionally substituted by lower alkyl, a benzyl group, optionally substituted by fluorine or chlorine, or 2-chloro-5-pyridylmethyl group, and
n represents 1.

4) Process for the preparation of 5-nitro-tetrahydropyrimidines of the general formula (I)

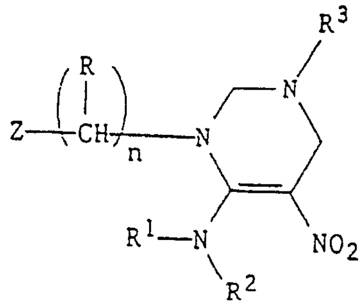

(I)

wherein

Z represents a substituted phenyl group or an optionally substituted five-membered or six-membered heterocyclic group comprising at least one hetero atom selected from the group consisting of nitrogen atom, oxygen atom and sulfur atom,

R represents hydrogen atom or a lower alkyl group,

$R^1$ and $R^2$ each represent hydrogen atom, an optionally substituted lower alkyl group, a lower alkenyl group, a lower alkynyl group, an optionally substituted phenyl group, an optionally substituted benzyl group,

$R^3$ represents a hydrogen atom, an optionally substituted alkyl group, a lower alkenyl group, a lower alkynyl group, a cycloalkyl group optionally substituted by a lower alkyl group, an optionally substituted phenyl group, an optionally substituted benzyl group, an optionally substituted heterocyclic group, and

n represents 0 or 1,

characterised in that

a) a compound represented by the general formula

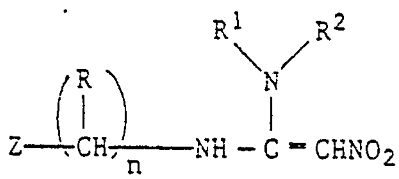

(II)

wherein

Z, R, $R^1$ and $R^2$ have the meanings mentioned above, is reacted with formaldehyde and a compound represented by the general formula

$NH_2$-$R^3$ (III)

wherein

$R^3$has the meaning mentioned above, or

b) a compound represented by the general formula (IV)

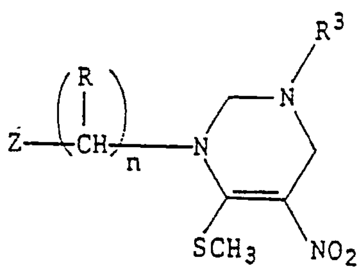

(IV)

wherein

Z, R, $R^3$ and n have the meanings mentioned above

is reacted with a compound of the general formula (V)

$NHR^1R^2$ (V)

wherein

$R^1$ and $R^2$ have the meanings mentioned above.

5) Insecticidal compositions, characterized in that they contain at least one 5-nitro-tetrahydropyrimidine

compound of the general formula (I).

6) Process for combating harmful insects, characterized in that 5-nitro-tetrahydropyrimidine compounds of the formula (I) are allowed to act on harmful insects and/or their habitat.

7) Use of 5-nitro-tetrahydropyrimidine compounds of the formula (I) for combating harmful insects.

8) Process for the preparation of insecticidal compositions characterized in that 5-nitro-tetrahydropyrimidine compounds of the formula (I) are mixed with extenders and/or surface active agents.

European Patent Office

# EUROPEAN SEARCH REPORT

Application number

EP 90110452.1

| Category | DOCUMENTS CONSIDERED TO BE RELEVANT<br>Citation of document with indication, where appropriate, of relevant passages | Relevant to claim | CLASSIFICATION OF THE APPLICATION (Int Cl 5) |
|---|---|---|---|
| X | CHEMICAL ABSTRACTS, vol. 87, no. 7, August 15, 1977, Columbus, Ohio, USA SONE, MASAKATSU et al. "Reaction of 1-nitro-2,2-bis-(methylthio)ethylene. V. Reaction with amines" page 464, column 2, abstract--no. 53 195v & Yakugaku Zasshi 1977, 97(3) 262-7 -- | 1 | C 07 D 401/06<br>C 07 D 401/04<br>C 07 D 403/04<br>C 07 D 403/06<br>C 07 D 239/06<br>A 01 N 43/54 |
| A | CHEMICAL ABSTRACTS, vol. 98, no. 29, May 9, 1983, Columbus, Ohio, USA VAN DER PLAS, HENK C. et al. "The delta adducts of 5-nitro-pyrimidines with liquid ammonia and their oxidation into aminonitropyridimides" page 448, column 1, abstract-no. 159 919b & J. Org. Chem. 1983, 48(8), 1354-7 -- | 1 | TECHNICAL FIELDS SEARCHED (Int Cl 5)<br>C 07 D 401/00<br>C 07 D 403/00<br>C 07 D 239/00 |
| A | CHEMICAL ABSTRACTS, vol. 104, no. 3, January 20, 1986, Columbus, Ohio, USA HASHIZUME, KIYOMATSU et al. "Model experiments on surugatoxin synthesis. III. Synthesis of 2,4-dibenzyloxy-6-ethylsulfonyl-5-nitro-pyrimidine" page 468, column 2, abstract-no.19 434g & Yakugaku Zasshi 1985, 105(4), 362-7 ---- | 1 | |

The present search report has been drawn up for all claims

| Place of search | Date of completion of the search | Examiner |
|---|---|---|
| VIENNA | 11-09-1990 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82